# EUROPÄISCHE PATENTSCHRIFT

(11) **EP 3 820 524 B1**
(45) Veröffentlichungstag und Bekanntmachung des Hinweises auf die Patenterteilung: **06.09.2023**
(21) Anmeldenummer: 19736763.4
(22) Anmeldetag: 12.07.2019
(51) Int. Cl.: A61K 47/58, A61K 41/00, A61P 35/00

(54) **HYPERICIN-PVP KOMPLEX MIT HOHEM HYPERICINANTEIL**
HYPERICIN-PVP COMPLEX WITH HIGH HYPERICIN CONTENT
COMPLEXE D'HYPERICINE PVP À PROPORTION DE L'HYPERICINE ÉLEVÉE

(30) Priorität: 13.07.2018 EP 18183435
(43) Veröffentlichungstag der Anmeldung: 19.05.2021
(73) Patentinhaber: Hypericum Lifescience GmbH, 1120 Wien (AT)
(72) Erfinder: KUBIN, Andreas, 1120 Wien (AT)
(74) Vertreter: SONN Patentanwälte OG
(86) Internationale Anmeldenummer: PCT/EP2019/068775
(87) Internationale Veröffentlichungsnummer: WO 2020/011960

(56) Entgegenhaltungen:
- WO-A1-2017/054017
- WO-A1-2017/054018
- WO-A2-01/89576
- A Kubin ET AL: "How to make hypericin water-soluble", Die Pharmazie, 1. April 2008 (2008-04-01), Seiten 263-269, XP055538699, Germany DOI: 10.1691/ph.2008.7292 Gefunden im Internet: URL:http://docserver.ingentaconnect.com/de liver/connect/govi/00317144/v63n4/s3.pdf?e xpires=1546867528&id=0000&titleid=75007325 &checksum=61F0A9D02FF3F8BEC2A47220AA01CC69
- DANIELA FEINWEBER ET AL: "Applicability of new degradable hypericin-polymer-conjugates as photosensitizers: principal mode of action demonstrated by in vitro models", PHOTOCHEMICAL AND PHOTOBIOLOGICAL SCIENCES, Bd. 13, Nr. 11, 1. Januar 2014 (2014-01-01), Seiten 1607-1620, XP055286452, GB ISSN: 1474-905X, DOI: 10.1039/C4PP00251B
- FREYTAG W E: "Determination of hypericin and detection of pseudohypericin in Hypericum perforatum by high-pressure liquid chromatography", DEUTSCHE APOTHEKER ZEITUNG, DT. APOTHEKER-VERLAG, DE, Bd. 124, Nr. 46, 1. Januar 1984 (1984-01-01), Seiten 2383-2386, XP009510279, ISSN: 0011-9857

## Beschreibung

Die vorliegende Erfindung betrifft einen Komplex aus Hypericin bzw. einem Hypericinsalz und Polyvinylpyrrolidon (PVP), welcher Komplex einen besonders hohen Anteil an Hypericin aufweist wie in den Ansprüchen definiert.

Hypericin ist als Inhaltsstoff in verschiedenen Pflanzen, besonders in Hypericum sp. und als Pigment in Protozen, in einigen australischen Insekten und mit Seitenketten als Fagopyrin im Buchweizen zu finden.

Wegen der photodynamischen und photochemischen Eigenschaften steht Hypericin immer wieder im Mittelpunkt verschiedener Forschungsprojekte, welche die Anwendung des Photosensitizers in der Tumordiagnostik und Tumortherapie untersuchen.

Die photodynamische Therapie (PDT) von Tumoren hat sich in den letzten 50 Jahre eher als Randgebiet der Onkologie entwickelt. Die Mechanismen der PDT sind jedoch weitgehend aufgeklärt und die Methoden definiert. Nach systemischer oder lokalen Applikation eines Photosensitizers erfolgt eine Akkumulation des Photosensitizers im malignen Gewebe. Wenn der Photosensitizer mithilfe von Licht geeigneter Wellenlänge angeregt wird, kann er Energie auf einen Reaktionspartner übertragen, z.B. auf molekularen Sauerstoff. Die dabei generierten reaktiven Sauerstoffmoleküle können wiederum die zellulären Strukturen des Tumorgewebes schädigen.

Bislang fehlt es aber an wirklich geeigneten Photosensitizern, die sich in Tumorzellen anreichern, gut verträglich sind und den physikalischen und chemischen Anforderungen der PDT entsprechen. Vor allem die Methode mittels Delta-Aminolävulinsäure (5-ALA) als "Prodrug" für Protoporphyrin IX in Hohlorganen (Magen, Darm, Blase, Lunge etc.) ist noch am ehesten forciert worden. Der Nachteil von 5-ALA bzw. Protoporphyrin IX, ist die Instabilität: die Porphyrine sind lichtempfindlich und bleichen während der Therapie aus, verlieren an Konzentration im Gewebe und sind bezüglich der Dosierung schwer kalkulierbar.

Die Stabilität von Hypericin als Photosensitizer ist bekannt, auch dass sich dieser Pflanzeninhaltsstoff in Tumorzellen anreichert. Das sind neben den chemischen und physikalischen Eigenschaften die optimalen Voraussetzungen für den Einsatz eines Sensitizers in der PDT. Durch *in vitro* Untersuchungen konnte die Wirksamkeit von Hypericin in der PDT in einer Reihe von Zelllinien bereits gezeigt werden und auch *in vivo* Tierstudien bestätigen das Potential von Hypericin in der PDT.

Hypericin ist eine hydrophobe Substanz, die in Wasser völlig unlöslich ist und verschiedenste Formulierungen braucht, um für medizinische Zwecke eingesetzt werden zu können. Solche Formulierungen enthalten dann verschiedene Lösungsmittel, die aber oft nicht gut verträglich sind und Nebenwirkungen zeigen (zB. Alkohol, DMSO etc.) oder Lösungsvermittler, Liposome, Mizellen oder Nanopartikel.

Die WO 01/89576 A2 beschreibt erstmals einen praktikablen Ansatz, Hypericin durch die Komplexierung mit Polyvinylpyrrolidon (PVP) wasserlöslich und damit applikationsfähig zu machen. Es werden darin Komplexe geoffenbart, die ein molares Verhältnis von Hypericin zu PVP von etwa 1:1 haben. Auf Seite 3 wird offenbart, dass Hypericin und PVP beide in einer Konzentration von 1 µmol/L vorliegen können. Gemäß dem laut der Anmeldung bevorzugten Molmassenbereich von PVP (10.000 - 90.000 g/mol) und der molaren Masse von Hypericin (504,44 g/mol) entspräche das einem Masseanteil von Hypericin am Hypericin-PVP Komplex zwischen 0,6 und 5 Gew.-%.

Kubin et. al. (Pharmazie 63 (2008) 263-269) beschreiben ein auf der WO 01/89576 A2 basiertes Herstellungsverfahren, in dem Hypericin, vorgelöst in Ethanol, mit PVP und Wasser auf 70°C erwärmt wird und die so erhaltene Lösung anschließend verdampft wird. Der erhaltene Rückstand enthält dann den wasserlöslichen Komplex Hypericin-PVP. In Abbildung 8 wird eine Lösung mit einer Konzentration von 50 µmol/L Hypericin und 100 µmol/L PVP (PVP 10, PVP 25, oder PVP 40) offenbart. Aus den molaren Massen von Hypericin (504,44 g/mol) und von PVP (10.000 g/mol für PVP 10, 25.000 g/mol für PVP 25, 40.000 g/mol für PVP 40) lassen sich die Molverhältnisse in Masseanteile umrechnen. Dabei erhält man für den Komplex mit PVP 10 einen Wert von etwa 2,5 Gew.-%, für PVP 25 einen Wert von etwa 1 Gew.-% und für PVP 40 einen Wert von etwa 0,6 Gew.-%.

Kubin et. al. (Photochemistry Photobiology 84 (2008) 1560-1563) beschreiben eine klinische Studie, in der Hypericin-PVP verwendet wurde. Hypericin hatte hier ebenfalls einen Masseanteil von 1 Gew.-% am Hypericin-PVP Komplex.

Die WO 2014/079972 A1 beschreibt Vorrichtungen für die PDT in Hohlorganen wie der Blase und erwähnt unter anderem die Verwendung von Hypericin-PVP. Als Gesamtmenge werden 0,25 mg Hypericin gebunden an 25 mg PVP angegeben. Der Masseanteil von Hypericin am Hypericin-PVP Komplex beträgt somit ebenfalls höchstens 1 Gew.-%.

Die WO 2017/054017 A1 beschreibt Formulierungen von Hypericin in der Form eines Salzes zur photodynamischen Therapie. In dem in Beispiel 1 beschriebenen Herstellungsverfahren von Hypericin-PVP werden 250,0 g einer Phosphatpufferlösung hergestellt, die insgesamt 1875 mg PVP k25 und 0,0225 mg Hypericin pro Gramm Lösung enthält. Der Masseanteil von Hypericin am Hypericin-PVP Komplex beträgt also 0,3 Gew.-%.

Die WO 2017/054018 A1 beschreibt Formulierungen von Hypericin in der Form eines Salzes zur photodynamischen Diagnose. In dem in Beispiel 1 beschriebenen Herstellungsverfahren von Hypericin-PVP werden 250,0 g einer Phosphatpufferlösung hergestellt, die insgesamt 562,5 mg PVP k25 und 0,0225 mg Hypericin pro Gramm Lösung enthält. Der Masseanteil von Hypericin am Hypericin-PVP Komplex beträgt hier also 1 Gew.-%.

Feinweber et al. (Photochemical & Photobiological Sciences 13.11 (2014): 1607-1620) beschreiben Konjugate bestehend aus Hypericin und hydrolytisch degradierbaren Polyphosphazenen. Zur Herstellung nicht-kovalenter Konjugate aus Hypericin und polydi[2-(2-oxo-1-pyrrolidinyl)ethoxy]-phosphazen (PYRP) wird Hypericin (2,4 mg) in 2 mL Ethanol gelöst und zu PYRP (200 mg) zugegeben, worauf das Lösungsmittel unter Vakuum entfernt wird. Zum Vergleich wird Hypericin-PVP herangezogen, wobei dieser Hypericin-PVP Komplex unter Verwendung von PVP40 "auf analoge Weise" hergestellt. Der Masseanteil von Hypericin am Hypericin-PVP Komplex beträgt demnach höchstens 1,2 Gew.-%.

Die photodynamische Diagnose mithilfe von Hypericin-PVP in Verbindung mit Fluoreszenzendoskopie wie aus dem Stand der Technik bekannt ist eine sehr empfindliche Methode und kann mit sehr geringem Materialeinsatz durchgeführt werden. Wie mehrfach bereits veröffentlicht, werden beispielsweise in die Blase lediglich 0,25 mg Hypericin (gesamt 25 mg Hypericin-PVP Komplex enthaltend 1 Gew.-% Hypericin) in gelöster Form eingebracht. Diese Menge reicht aus, um die Tumore und Läsionen soweit anzufärben, dass sie vom Urologen identifiziert und entfernt werden können. Der geringe Masseanteil von Hypericin am Hypericin-PVP Komplex und der damit einhergehende hohe Anteil von PVP stellt aufgrund des niedrigen Materialeinsatzes daher kein Problem für die photodynamische Diagnose dar.

Dies gilt jedoch nicht für Anwendungen, die höhere Mengen von Hypericin benötigen, wie z.B. die photodynamische Therapie. Die höhere Dosierung von Hypericin bringt in diesem Fall eine hohe Menge von PVP mit sich. Es besteht daher ein Bedarf an Hypericin-PVP Komplexen, die einen höheren Anteil an Hypericin enthalten. Eine Aufgabe der vorliegenden Erfindung ist es daher, solche Komplexe zur Verfügung zu stellen.

Gelöst wird diese Aufgabe durch einen Komplex aus Hypericin bzw. einem Hypericinsalz und Polyvinylpyrrolidon (PVP), wobei der mittlere Masseanteil von Hypericin bzw. vom Hypericinsalz am Gesamtkomplex größer ist als 6 Gew.-%.

Wenn in der Folge die Rede von "Hypericin" ist, soll dieser Begriff sowohl die freie Säure als auch die Salze von Hypericin, vorzugsweise die Alkalisalze, besonders bevorzugt das Natriumoder Kaliumsalz, mit einschließend verstanden werden.

Mit dem erfindungsgemäßen Hypericin-PVP Komplex können wasserlösliche Zusammensetzungen bereitgestellt werden, die große Mengen an Hypericin enthalten, ohne dass damit eine unvorteilhaft große Menge an PVP mit einhergeht. Formulierungen können damit auch materialsparender als bisher zusammengestellt werden. Dadurch, dass große Mengen an Adjuvantien (PVP) und damit einhergehende Nebenwirkungen vermieden werden können, erleichtert die vorliegende Erfindung Anwendungen, die größere Mengen an Hypericin benötigen. Dies betrifft insbesondere Therapieverfahren, wie photodynamischen Therapie (PDT) zur Behandlung von Tumorerkrankungen.

Die vorliegende Erfindung stellt daher auch pharmazeutische Zusammensetzungen umfassend den erfindungsgemäßen Hypericin-PVP Komplex zur Verfügung.

Bei den durchgeführten experimentellen Studien im Zuge der vorliegenden Erfindung hat sich unerwartet gezeigt, dass ein besonders hoher Masseanteil von Hypericin am Hypericin-PVP Komplex dadurch erreicht werden kann, dass ein Gemisch von Hypericin und PVP auf eine Temperatur erhitzt wird, die über der Glasübergangstemperatur des eingesetzten PVPs liegt.

Die vorliegende Erfindung stellt daher auch ein Verfahren zur Herstellung des erfindungsgemäßen Komplexes zur Verfügung, dass dadurch gekennzeichnet ist, dass ein Gemisch von Hypericin und PVP auf eine Temperatur erhitzt wird, die über der Glasübergangstemperatur des eingesetzten PVPs liegt.

Durch Anwendung dieses Verfahrens konnten die Erfinder Hypericin-PVP Komplexe herstellen, die mehr als 6 Gew.-%, insbesondere mehr als 10 Gew.-%, bevorzugt mehr als 15 Gew.-%, besonders bevorzugt mehr als 20 Gew.-% und am meisten bevorzugt mehr als 35 Gew.% Hypericin enthielten. Der Hypericinanteil der erfindungsgemäßen Komplexe übersteigt damit deutlich den Hypericinanteil der im Stand der Technik bekannten Hypericin-PVP Komplexe und ermöglicht dadurch neue und vorteilhaftere Anwendungen von Hypericin-PVP.

Besonders vorteilhaft ist die Anwendung der erfindungsgemäßen Hypericin-PVP Komplexe in der photodynamischen Therapie (PDT) zur Behandlung von Tumorerkrankungen.

Auch weitere Anwendungen, die größere Mengen an Hypericin verlangen, werden durch die vorliegende Erfindung erheblich erleichtert. Z.B, hat Hypericin in Verbindung mit Licht eine antivirale und antibakterielle Wirkung. Der erfindungsgemäße Hypericin-PVP Komplex kann somit für die Sterilisation und/oder Desinfektion von Oberflächen oder Flüssigkeiten verwendet werden. Der PVP Anteil bleibt bei einer Anwendung in diesem Sinne überschaubar gering.

Hypericin kann auch bezeichnet werden als 1,3,4,6,8,13-Hexahydroxy-10,11-dimethylphenanthro[1,10,9,8-opqra]perylen-7,14-dion. Hypericin kann durch die folgende Strukturformel dargestellt werden (hier in der Form der freien Säure):

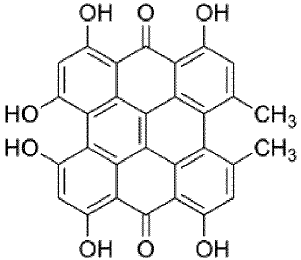

Hypericin kann neben der freien Säure auch in anderen Formen vorliegen, wie in der Form von Salzen, z.B. Alkalimetallsalzen wie Natrium- oder Kaliumsalzen. Der Begriff "Hypericin" bezeichnet im Rahmen der vorliegenden Erfindung alle möglichen solche Formen.

Polyvinylpyrrolidon (PVP), auch Polyvidon oder Povidon, ist ein Polymer der Verbindung Vinylpyrrolidon. Handelsüblich ist PVP in unterschiedlichen Polymerisationsgraden erhältlich. Der Polymerisierungsgrad bestimmt die mittlere molare Masse des Polymers.

Die vorliegende Erfindung betrifft einen Komplex aus Hypericin bzw. einem Hypericinsalz und Polyvinylpyrrolidon (PVP), dadurch gekennzeichnet, dass der mittlere Masseanteil von Hypericin bzw. vom Hypericinsalz am Gesamtkomplex größer ist als 6 Gew.-%, vorzugsweise größer ist als 8 Gew.-%, 10 Gew.-%, 15 Gew.-%, 20 Gew.-%, 25 Gew.-%, bzw. als 30 Gew.-%.

Prozentangaben (%) beziehen sich in der vorliegenden Erfindung wenn nicht anders angegeben jeweils auf Gewichtsprozent (Gew.-%). Der Masseanteil gibt hier den relativen Anteil der Masse von Hypericin an der Masse des Gesamtkomplexes (Hypericin + PVP) wieder.

Der Anteil von Hypericin am Hypericin-PVP Komplex kann auch als Stoffmengenverhältnis ausgedrückt werden. Beispielsweise hat ein Hypericin-PVP Komplex, der aus Hypericin in der Form der freien Säure (molare Masse: 504,44 g/mol) und PVP mit einer mittleren molaren Masse von 25 kD besteht, und der einen mittleren Masseanteil von Hypericin am Gesamtkomplex von 10 Gew.-% aufweist, ein mittleres Stoffmengenverhältnis von Hypericin zu PVP im Komplex von 5,5. Der mittlere Stoffmengenanteil gibt hier das Verhältnis der Stoffmenge von Hypericin zur Stoffmenge von PVP wieder.

Die vorliegende Erfindung betrifft daher auch einen Komplex aus Hypericin und PVP, dadurch gekennzeichnet, dass das mittlere Stoffmengenverhältnis von Hypericin zu PVP im Komplex größer ist als 2,5, vorzugsweise größer ist als 3, 4, 5, 7, 10, bzw. 15.

Der Anteil (Masseanteil wie auch Stoffmengenverhältnis) von Hypericin am Gesamtkomplex ist hierin stets als mittlerer Anteil zu verstehen. Eine geeignete Methode, um den mittleren Anteil von Hypericin am Gesamtkomplex zu bestimmen, ist die Hochleistungsflüssigkeitschromatographie (englisch high performance liquid chromatography, HPLC). Der Fachmann ist mit der Durchführung einer solchen Bestimmung, wie beschrieben in Freytag W.E. (Deutsche Apothekerzeitung 124 Nr. 46 (1984) 2383-2386), vertraut. Dem Fachmann ist bekannt, wie er eine genaue Menge des Hypericin-PVP Komplexes einwiegen kann, wie er mittels einer Kalibrationsgerade und einer HPLC-Messung die Konzentration von Hypericin bestimmen kann und wie er daraus den Masseanteil von Hypericin am Gesamtkomplex errechnen kann. Ein Beispiel, wie eine solche Bestimmung durchgeführt werden kann, ist in Beispiel 5 beschrieben.

Handelsübliches PVP ist in einer Vielzahl unterschiedlicher Polymerisierungsgrade und damit mittlerer molarer Massen erhältlich. Bevorzugt werden im Rahmen der vorliegenden Erfindung vor allem molare Massen im Bereich von 10 kD bis 40 kD, weil Moleküle in dieser Größe noch gut und unmetabolisiert von der Niere ausgeschieden werden können (Clearance).

In einer bevorzugten Ausführungsform der vorliegenden Erfindung ist der Hypericin-PVP Komplex daher dadurch gekennzeichnet, dass das PVP eine mittlere molare Masse von 10 bis 40 kD, vorzugsweise von 12 bis 25 kD, hat.

Die bisher im Stand der Technik beschriebenen Hypericin-PVP Komplexe eignen sich aufgrund des niedrigen Anteils von Hypericin nicht gut als Inhaltsstoffe pharmazeutischer Zusammensetzungen. Um ausreichend große Mengen von Hypericin für pharmazeutische Anwendungen zu erreichen, müssten große Mengen des Hypericin-PVP Komplexes eingesetzt werden. Dies würde einen unvorteilhaft hohen Materialverbrauch bzw. Akkumulierung von PVP im Patienten zur Folge haben. Mit der vorliegenden Erfindung wird dieses Problem gelöst. Durch den stark reduzierten Anteil an Adjuvantien (PVP), sind höhere Dosierungen von wasserlöslichem Hypericin möglich, die für die Tumortherapie erforderlich sind. Höhere Nebenwirkungen von PVP können dadurch verhindert werden. Die neue Erfindung ermöglicht somit unter anderem den Einsatz von Hypericin in der photodynamischen Tumortherapie.

Die vorliegende Erfindung betrifft daher auch eine pharmazeutische Zusammensetzung umfassend den erfindungsgemäßen Hypericin-PVP Komplex.

In einer bevorzugten Ausführungsform der Erfindung ist die pharmazeutische Zusammensetzung dadurch gekennzeichnet, dass die Zusammensetzung den erfindungsgemäßen Hypericin-PVP Komplex mit Hypericin in einer Konzentration von mindestens 25 mg/L, vorzugsweise mindestens 50 mg/L, 75 mg/L, 100 mg/L, 150 mg/L, bzw. mindestens 250 mg/L enthält.

Die pharmazeutischen Zusammensetzungen im Sinne der vorliegenden Erfindung können beispielsweise zur Behandlung von Tumoren lokal verabreicht werden. Dadurch, dass durch die vorliegende Erfindungen erstmals pharmazeutische Zusammensetzungen hergestellt werden können, die hohe Konzentrationen von Hypericin enthalten, ohne dass große Mengen an PVP eingesetzt werden müssen, ermöglicht die vorliegende Erfindung auch pharmazeutische Zusammensetzungen, die intravenös verabreicht werden können. So können große Mengen an Hypericin, das sich in der Folge in Tumorzellen anreichert, auch systemisch verabreicht werden.

In einer weiteren bevorzugten Ausführungsform betrifft die vorliegende Erfindung daher eine pharmazeutische Zusammensetzung enthaltend den erfindungsgemäßen Hypericin-PVP Komplex, dadurch gekennzeichnet, dass die Zusammensetzung zur intravenösen Verabreichung vorgesehen ist.

In einer besonders bevorzugen Ausführungsform betrifft die vorliegende Erfindung eine pharmazeutische Zusammensetzung enthaltend den erfindungsgemäßen Hypericin-PVP Komplex, dadurch gekennzeichnet, dass die Zusammensetzung zur intravenösen Verabreichung vorgesehen ist und dass die Zusammensetzung Hypericin in einer Konzentration von mindestens 25 mg/L, vorzugsweise mindestens 50 mg/L, 60 mg/L, 80 mg/L, 100 mg/L, 150 mg/L bzw. mindestens 250 mg/L enthält.

PVP hat als amorphe Substanz keinen Schmelzpunkt, sondern zeigt eine sogenannte Glasübergangstemperatur. Die Glasübergangstemperatur ist unter anderem vom Polymerisierungsgrad, also von der mittleren molaren Masse, von PVP abhängig (siehe Tabelle 1) .

**Tabelle 1: Beispiele für die Glasübergangstemperatur von**

| PVP molare Masse | Glasübergangstemperatur |
|---|---|
| 12 kD | 93 °C |
| 17 kD | 130 °C |
| 25 kD | 155 °C |
| 30 kD | 175 °C |

*PVP in Abhängigkeit der molaren Masse.*

Bei den im Zuge der vorliegenden Erfindung angestellten experimentellen Studien hat sich unerwartet gezeigt, dass ein besonders hoher Masseanteil von Hypericin am Hypericin-PVP Komplex dadurch erreicht werden kann, dass ein Gemisch von Hypericin und PVP auf eine Temperatur erhitzt wird, die über der Glasübergangstemperatur des eingesetzten PVPs liegt. Hypericin ist bis etwa 300°C stabil und kann somit als intaktes Molekül an PVP komplexiert werden. Diese Methode ermöglicht somit die Herstellung des erfindungsgemäßen Hypericin-PVP Komplexes.

Die vorliegende Erfindung betrifft daher auch ein Verfahren zur Herstellung des erfindungsgemäßen Hypericin-PVP Komplexes, dadurch gekennzeichnet, dass ein Gemisch von Hypericin und PVP auf eine Temperatur erhitzt wird, die über der Glasübergangstemperatur des eingesetzten PVPs liegt.

Als "Glasübergangstemperatur des eingesetzten PVPs" soll in diesem Zusammenhang die Temperatur verstanden werden, bei der der Glasübergang von PVP im vorliegenden Gemisch mit Hypericin erfolgt. Die Glasübergangstemperatur des eingesetzten PVPs kann von der Zusammensetzung des Gemisches beeinflusst werden, beispielsweise wenn der Mischung Lösungsmittel oder Wasser zugesetzt werden. Methoden zur Bestimmung der Glasübergangstemperatur sind dem Fachmann bekannt. Vorzugsweise kann die Glasübergangstemperatur dem Verfahren der entsprechenden DIN-Norm folgend bestimmt werden.

Eine bevorzugte Ausführungsform des erfindungsgemäßen Verfahrens zur Herstellung des Hypericin-PVP Komplexes ist dadurch gekennzeichnet, dass die molare Masse des eingesetzten PVPs bei mindestens 12 kD liegt und die Mischung auf eine Temperatur von mindestens 93°C erhitzt wird.

Eine besonders bevorzugte Ausführungsform ist dadurch gekennzeichnet, dass die molare Masse des eingesetzten PVPs bei mindestens 17 kD liegt und die Mischung auf eine Temperatur von mindestens 130°C erhitzt wird.

Eine weitere besonders bevorzugte Ausführungsform ist dadurch gekennzeichnet, dass die molare Masse des eingesetzten PVPs bei mindestens 25 kD liegt und die Mischung auf eine Temperatur von mindestens 155°C erhitzt wird.

Eine weitere besonders bevorzugte Ausführungsform ist dadurch gekennzeichnet, dass die molare Masse des eingesetzten PVPs bei mindestens 35 kD liegt und die Mischung auf eine Temperatur von mindestens 175°C erhitzt wird.

Es hat sich als vorteilhaft erwiesen, dem Gemisch von Hypericin und PVP ein Lösungsmittel oder ein Gemisch von Lösungesmitteln zuzufügen. Bevorzugterweise wird das Gemisch von Hypericin und PVP in wenig Lösungsmittel pastös verrührt. Zusätzliche Lösungsmittel können dazu beitragen, dass sich die Komponenten homogen verteilen und sich größere Mengen an Hypericin an PVP anlagern können. Als Lösungsmittel eignen sich z.B. Wasser, Ethanol, Methanol, Pyridin, Aceton, Ethylmethylketon und Pyridin, bzw. Mischungen davon. Besonders bevorzugt werden Wasser, Ethanol, Methanol und Pyridin, vor allem Wasser und Ethanol.

Demgemäß ist eine bevorzugte Ausführungsform des erfindungsgemäßen Herstellungsverfahren des Hypericin-PVP Komplexes dadurch gekennzeichnet, dass dem Gemisch von Hypericin und PVP ein Lösungsmittel oder ein Gemisch von Lösungsmitteln, vorzugsweise Wasser, Ethanol, Methanol, Pyridin, Aceton, Ethylmethylketon, und/oder Ethylacetat, noch mehr bevorzugt Wasser, Ethanol, Methanol, und/oder Pyridin, noch mehr bevorzugt Wasser und/oder Ethanol, zugefügt wird.

Es hat sich im Herstellungsverfahren als zweckmäßig erwiesen, das Gemisch von Hypericin und PVP für einen bestimmten Zeitraum auf einer Temperatur, die über der Glasübergangstemperatur des eingesetzten PVPs liegt, zu halten. Gute Resultate wurden erzielt, wenn die Mischung für mindestens 5 Minuten über der Glasübergangstemperatur gehalten wurde.

Demgemäß ist eine weitere bevorzugte Ausführungsform des erfindungsgemäßen Herstellungsverfahren des Hypericin-PVP Komplexes dadurch gekennzeichnet, dass die Mischung für mindestens 5 Minuten auf einer Temperatur, die über der Glasübergangstemperatur des eingesetzten PVPs liegt, gehalten wird.

Die photodynamische Therapie (PDT) von Tumorerkrankungen hat sich in den letzten 50 Jahren eher als Randgebiet der Onkologie entwickelt. Die Mechanismen der PDT sind jedoch weitgehend aufgeklärt und die Methoden im Stand der Technik bekannt. Bislang fehlt es aber an gut geeigneten Photosensitizern, die sich in Tumorzellen anreichern, gut verträglich sind und den physikalischen und chemischen Anforderungen der PDT entsprechen. Die vorliegende Erfindung stellt Hypericin-PVP Komplexe zur Verfügung, die diesen Anforderungen entsprechen und die sich aufgrund des hohen Anteils von Hypericin hervorragend für therapeutische Anwendungen eignen. Die erfindungsgemäßen Hypericin-PVP Komplexe können dabei entweder lokal oder systemisch verabreicht werden.

Die vorliegende Erfindung betrifft daher auch pharmazeutische Zusammensetzungen enthaltend einen erfindungsgemäßen Hypericin-PVP Komplex zur Verwendung in einem Therapieverfahren, vorzugsweise zur Anwendung in der photodynamischen Therapie (PDT) zur Behandlung von Tumorerkrankungen.

Die vorliegende Erfindung betrifft auch ein Verfahren zur Behandlung von Krebserkrankungen, dadurch gekennzeichnet, dass ein erfindungsgemäßer Hypericin-PVP Komplex verabreicht wird, vorzugsweise im Zuge einer photodynamischen Therapie (PDT).

In einer bevorzugten Ausführungsform stellt die vorliegende Erfindung ein Verfahren zur Behandlung von Krebserkrankungen zur Verfügung, umfassend die Schritte:
- Bereitstellen einer pharmazeutisch annehmbaren Formulierung enthaltend einen erfindungsgemäßen Hypericin-PVP Komplex; und
- Verabreichen einer wirksamen Menge dieser Zusammensetzung an eine Person, die Krebs hat.

Vorzugsweise weist das Verfahren zur Behandlung von Krebserkrankungen als zusätzlichen Schritt das Bestrahlen der Person mit Licht. Das Licht hat vorzugsweise eine Wellenlänge zwischen 400 nm und 800 nm, insbesondere zwischen 500 nm und 700 nm, am meisten bevorzugt zwischen 550 nm und 650 nm. Das Licht hat vorzugsweise eine Intensität zwischen 1 mW/cm² und 250 mW/cm², besonders bevorzugt zwischen 2 mW/cm² und 100 mW/cm², noch mehr bevorzugt zwischen 3 mW/cm² und 50 mW/cm², am meisten bevorzugt zwischen 5 mW/cm² und 25 mW/cm².

Hypericin hat in Verbindung mit Licht eine antivirale und antibakterielle Wirkung. Somit kann der erfindungsgemäße Komplex auch für die Sterilisation und/oder Desinfektion von Oberflächen oder Flüssigkeiten eingesetzt werden. Der hohe Anteil von Hypericin am Hypericin-PVP Komplex ist dabei ein erheblicher Vorteil, weil somit der Verbrauch von PVP minimiert wird.

Die vorliegende Erfindung betrifft daher auch die Verwendung eines erfindungsgemäßen Hypericin-PVP Komplexes zur Sterilisation und/oder Desinfektion von Oberflächen oder Flüssigkeiten.

"Hypericin-PVP" bzw. "Hypericin-PVP Komplex" hierin bezieht sich auf ein Erzeugnis, das Hypericin und PVP enthält, und worin eine Verbindung zwischen Hypericin und PVP Molekülen besteht. Das Wort "Komplex" schränkt die Art der Verbindung in keiner Weise ein, sondern bedeutet lediglich, dass eine Verbindung zwischen einem oder mehreren Hypericin Molekülen und einem oder mehreren PVP Molekülen besteht. Die Verbindung kann z.B. eine nicht-kovalente Anlagerung von Hypericin an PVP sein. Als "Hypericin-PVP" bzw. "Hypericin-PVP Komplex" soll das Erzeugnis insgesamt und nicht ein einzelner Hypericin-PVP Komplex auf molekularer Ebene verstanden werden.

Die Bezeichnungen "Anteil am Komplex", "Masseanteil", "Stoffmengenverhältnis" hierin sind immer als mittlere Werte zu verstehen. Sie beziehen sich nicht auf jeden einzelnen Komplex auf molekularer Ebene sondern beziehen sich auf einen mittleren Wert des gesamten Erzeugnisses.

Die vorliegende Erfindung wird durch die folgenden Beispiele und die Figuren illustriert.

In den Figuren zeigen:

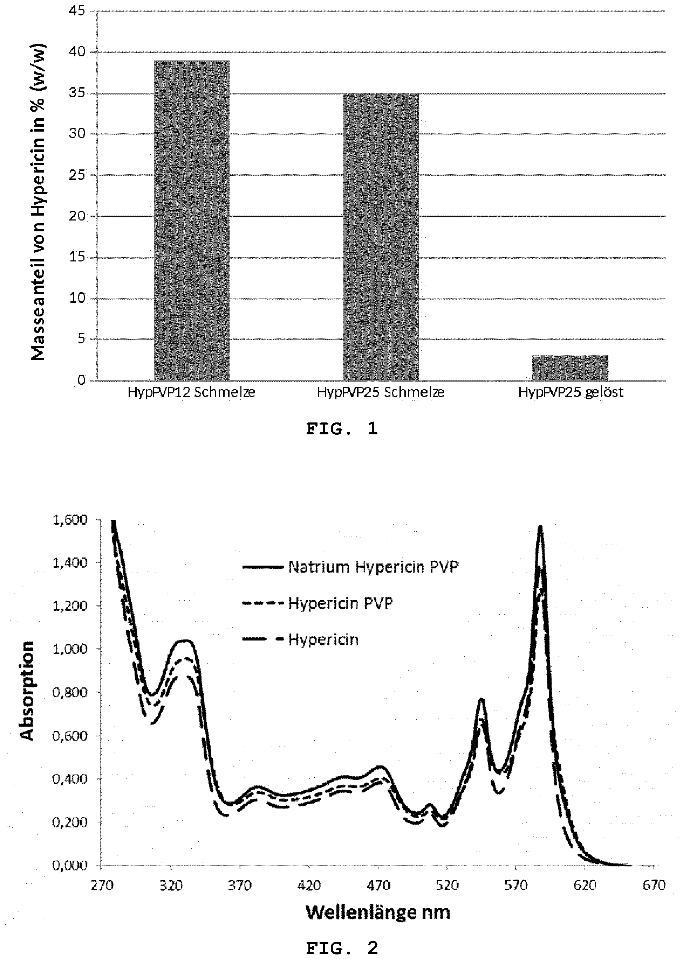

**Figur 1****:** Masseanteil von Hypericin am Gesamtkomplex in zwei erfindungsgemäßen Hypericin-PVP Komplexen im Vergleich zu einem herkömmlichen Hypericin-PVP Komplex. Beim für die erfindungsgemäßen Komplexe eingesetzten PVP handelt es sich um PVP mit einer mittleren molaren Masse von entweder 12 kD ("HypPVP12 Schmelze) oder 25 kD ("HypPVP25 Schmelze"). Beim für die Herstellung des herkömmlichen Komplexes eingesetzten PVP handelt es sich um PVP mit einer mittleren molaren Masse von 25 kD ("HypPVP25 gelöst"). Die erfindungsgemäßen Komplexe wurden hergestellt wie beschrieben in Beispiel 1. Der herkömmliche Komplex wurde hergestellt wie beschrieben in Beispiel 3.
**Figur 2****:** Vergleich der Absorptionsspektren von Natrium-Hypericinat-PVP, Hypericin-PVP und Hypericin. Die photophysikalischen Eigenschaften von Hypericin bleiben im PVP-Komplex erhalten, unabhängig davon, ob die freie Säure oder das Natriumsalz von Hypericin eingesetzt wird, oder Hypericin in komplexierter Form vorliegt. Das heißt, dass Hypericin in durch PVP komplexierter Form seine photophysikalischen Eigenschaften nicht verliert.

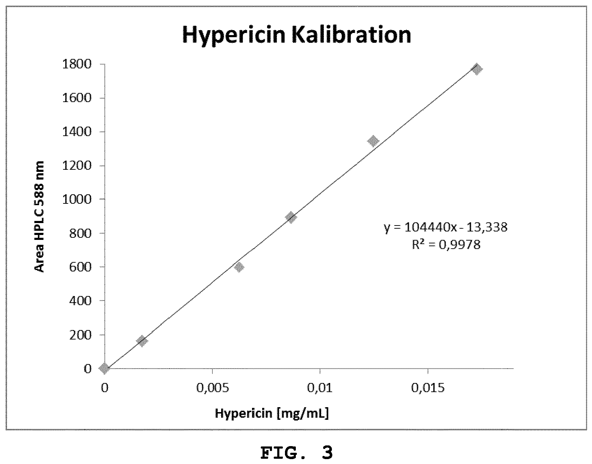

**Figur 3****:** Kalibrationsgerade zur Bestimmung des Masseanteils von Hypericin am Hypericin-PVP Komplex mittels HPLC. HPLC-Läufe wurde wie in Beispiel 5 beschrieben mit unterschiedlichen Mengen von Hypericin durchgeführt. Die Konzentration des eingewogenen Hypericins (x-Achse) wurde gegen die Fläche der Absorptionspeaks bei 588 nm aufgetragen und eine lineare Regression wurde erstellt.

### Beispiel 1 - Herstellung eines erfindungsgemäßen Hypericin-PVP Komplexes

30 mg Polyvinylpyrrolidon (PVP 25 kD) wurde mit 15 mg Hypericin (99 % HPLC) trocken vermischt. Zu dem Gemisch wurden 200 µL (200 Mikroliter sind gleich 0,2 mL) Ethanol zugefügt und 100 µL Wasser (100 Mikroliter sind gleich 0,1 mL). Dann wurde die Mischung verrührt und für 20 Minuten stehen gelassen. Danach wurde das Gemisch langsam im Trockenschrank auf 180°C erhitzt. Die Temperaturführung von Raumtemperatur auf 180°C wurde in einem Zeitraum von etwa 20 Minuten durchgeführt. Das Gemisch wurde für etwa 8 Minuten bei einer Temperatur von 180°C gehalten. Danach wurde das Gemisch auf Raumtemperatur abgekühlt. Anschließend wurden etwa 3-6 mL Wasser zugefügt und für 1 Stunde gerührt. Dabei gingen die löslichen Bestandteile, der Hypericin-PVP Komplex, in Lösung. Die unlöslichen Bestandteile und das nicht komplexierte Hypericin wurde abfiltriert (Porendurchmesser 0,2 - 0,4 µm) . Das Filtrat wurde getrocknet und trocken bis zur Weiterverwendung gelagert.

### Beispiel 2 - Herstellung eines erfindungsgemäßen Hypericin-PVP Komplexes, enthaltend Hypericin in der Form des Natriumsalzes

Wie Beispiel 1, jedoch wurde statt Hypericin das Natriumsalz verwendet: Na-Hypericinat. Na-Hypericinat wurde nach der Vorschrift von Kapinus et al. (Monatshefte für Chemie 130 (1999) 436-441) hergestellt.

### Beispiel 3 - Herstellung eines Hypericin-PVP Komplexes gemäß Stand der Technik

Ein herkömmlicher Hypericin-PVP Komplex wurde hergestellt wie beschrieben in Kubin et. al. "How to make hypericin water-soluble", Die Pharmazie 63 (2008) 263-269. Dazu wurden 10 mg Hypericin in 2,5 mL Ethanol unter Ultraschall gelöst, danach wurden 1000 mg PVP 25 kD und 8 mL destilliertes Wasser zugegeben. Das Gemisch wurde für ca. 5 Minuten auf 70 °C erhitzt. Danach wurden 5 mL Wasser zugegeben und das Gemisch für weitere 10 Minuten gerührt. Die Lösung wurde anschließen im Rotavapor getrocknet und der erhaltene Hypericin-PVP Komplex trocken bis zur Weiterverwendung gelagert.

Beim Versuch mehr Hypericin zu der Komplexierung hinzuzufügen ergab sich ein Niederschlag, da unter diesen Bedingungen keine Komplexierung von wesentlich größeren Mengen Hypericin durch PVP möglich ist.

### Beispiel 4 - Charakterisierung der Hypericin-PVP Komplexe

Hypericin-PVP Komplexe wurden gemäß den in Beispielen 1 bis 3 beschriebenen Verfahren hergestellt.

Der Masseanteil von Hypericin an den Hypericin-PVP Komplexen wurde mittels HPLC und der entsprechenden Kalibration von Hypericin bestimmt. Als Referenzmaterial wurde standardisiertes Hypericin (> 99 %, Firma Planta Naturstoffe VertriebsGmbH) verwendet. Die Bestimmung des Masseanteils von Hypericin am Gesamtkomplex wurde durchgeführt wie beschrieben in Beispiel 5.

Bei den erfindungsgemäßen Komplexen wurden Masseanteile von bis zu 40 Gew.-% (Hypericin freie Säure; Herstellung gern. Beispiel 1) bzw. 41 Gew.-% (Natriumhypericinat; Herstellung gern. Beispiel 2) gemessen, während der Masseanteil des herkömmlichen Komplexes bei 1 Gew.-% lag (Herstellung gern. Kubin et. al., Die Pharmazie 63 (2008) 263-269 wie beschrieben in Beispiel 3). Durch Einsatz des erfindungsgemäßen Verfahrens konnte PVP also mit der nahezu 40-fachen Menge an Hypericin beladen werden.

### Beispiel 5 - Bestimmung des Masseanteils von Hypericin am Gesamtkomplex

Der Masseanteil von Hypericin an den Hypericin-PVP Komplexen wurde mittels HPLC und der entsprechenden Kalibration mit Hypericin bestimmt. Die HPLC Methode wurde im Wesentlichen durchgeführt wie beschrieben in Freytag W.E. (Deutsche Apothekerzeitung 124 Nr. 46 (1984) 2383-2386).

Im Detail wurde die HPLC Methode durchgeführt mit:
- Laufmittel: 568,0 g Methanol, 157,8 g Ethylacetat, 185,5 g Puffer (13,8 g NaH₂PO₄H2O in 1000 ml aqua dest. mit 85% Orthophosphorsäure auf pH = 2,1)
- Säule: Nucleosil 120 3C18 (120 mm Länge, 4 mm Innendurchmesser)
- Flußrate: 0,6 mL/min
- Detektion: UV-Vis bei 588 nm

Für die Kalibration wurde standardisiertes Hypericin (> 99 %, Firma Planta Naturstoffe Vertriebs GmbH) als Referenzmaterial verwendet. HPLC-Läufe mit unterschiedliche Mengen des Referenzmaterials gelöst im HPLC Laufmittel wurden durchgeführt und die Fläche der Hypericin-Absorptionspeaks bei 588 nm wurde bestimmt. Aus den gemessenen Werten wurde durch lineare Regression eine Kalibrationsgerade erstellt (siehe Figur 3).

Zur Bestimmung des Hypericin-Anteils eines gemäß Beispiel 1 hergestellten erfindungsgemäßen Hypericin-PVP Komplexes wurden aus dem getrockneten Hypericin-PVP Komplex (Pulver) genau 5 mg eingewogen und im HPLC Laufmittel gelöst. Ein HPLC-Lauf wurde wie oben beschrieben durchgeführt und aus dem erhaltenen Absorptionssignal bei 588 nm wurde anhand der zuvor erstellten Kalibrationsgerade der Anteil von Hypericin am Gesamtkomplex bestimmt. Als Resultat wurde 40 Gew.-% erhalten (d.h. in 100 mg Hypericin-PVP Komplex waren beispielsweise 40 mg Hypericin enthalten und 60 mg PVP).

## Patentansprüche

1. Komplex aus Hypericin bzw. einem Hypericinsalz und Polyvinylpyrrolidon (PVP), **dadurch gekennzeichnet, dass** der mittlere Masseanteil von Hypericin bzw. vom Hypericinsalz am Gesamtkomplex größer ist als 6 Gew.-%.

2. Komplex gemäß Anspruch 1, **dadurch gekennzeichnet, dass** das mittlere Stoffmengenverhältnis von Hypericin zu PVP im Komplex größer ist als 2,5.

3. Komplex gemäß Anspruch 1 oder 2, **dadurch gekennzeichnet, dass** das PVP eine mittlere molare Masse von 10 bis 40 kD, vorzugsweise von 12 bis 25 kD, hat.

4. Pharmazeutische Zusammensetzung enthaltend einen Komplex gemäß einem der Ansprüche 1 bis 3.

5. Pharmazeutische Zusammensetzung gemäß Anspruch 4, **dadurch gekennzeichnet, dass** die Zusammensetzung zur intravenösen Verabreichung vorgesehen ist und dass die Zusammensetzung Hypericin in einer Konzentration von mindestens 25 mg/L enthält.

6. Verfahren zur Herstellung eines Komplexes gemäß einem der Ansprüche 1 bis 3, **dadurch gekennzeichnet, dass** ein Gemisch von Hypericin und PVP auf eine Temperatur erhitzt wird, die über der Glasübergangstemperatur des eingesetzten PVPs liegt.

7. Verfahren gemäß Anspruch 6, **dadurch gekennzeichnet, dass** dem Gemisch von Hypericin und PVP ein Lösungsmittel oder ein Gemisch von Lösungsmitteln, vorzugsweise Wasser, Ethanol, Methanol, Pyridin, Aceton, Ethylmethylketon, und/oder Ethylacetat, noch mehr bevorzugt Wasser, Ethanol, Methanol, und/oder Pyridin, noch mehr bevorzugt Wasser und/oder Ethanol, zugefügt wird.

8. Verfahren gemäß Anspruch 6 oder 7, **dadurch gekennzeichnet, dass** die Mischung für mindestens 5 Minuten auf einer Temperatur, die über der Glasübergangstemperatur des eingesetzten PVPs liegt, gehalten wird.

9. Pharmazeutische Zusammensetzung gemäß Anspruch 4 oder 5 zur Verwendung in einem Therapieverfahren, vorzugsweise zur Anwendung in der photodynamischen Therapie (PDT) zur Behandlung von Tumorerkrankungen.

10. Verwendung eines Komplexes gemäß einem der Ansprüche 1 bis 3 zur Sterilisation und/oder Desinfektion von Oberflächen oder Flüssigkeiten.

## Claims

1. A complex formed from hypericin or a hypericin salt and polyvinylpyrrolidone (PVP), **characterized in that** the mean proportion by weight of hypericin or of hypericin salt with respect to the total complex is more than 6 % by weight.

2. The complex as claimed in claim 1, **characterized in that** the mean molar ratio of hypericin to PVP in the complex is more than 2.5.

3. The complex as claimed in claim 1 or claim 2, **characterized in that** the PVP has a mean molar mass of 10 to 40 kD, preferably from 12 to 25 kD.

4. A pharmaceutical composition containing a complex as claimed in one of claims 1 to 3.

5. The pharmaceutical composition as claimed in claim 4, **characterized in that** the composition is provided for intravenous administration and **in that** the composition contains hypericin in a concentration of at least 25 mg/L.

6. The method for the production of a complex as claimed in one of claims 1 to 3, **characterized in that** a mixture of hypericin and PVP is heated to a temperature which is above the glass transition temperature of the PVP employed.

7. The method as claimed in claim 6, **characterized in that** a solvent or a mixture of solvents, preferably water, ethanol, methanol, pyridine, acetone, ethylmethyl ketone and/or ethyl acetate, more preferably water, ethanol, methanol and/or pyridine, more preferably water and/or ethanol, is added to the mixture of hypericin and PVP.

8. The method as claimed in claim 6 or claim 7, **characterized in that** the mixture is maintained at a temperature which is above the glass transition temperature of the PVP employed for at least 5 minutes.

9. A pharmaceutical composition as claimed in claim 4 or claim 5, for use in a therapeutic method, preferably for application in photodynamic therapy (PDT) for the treatment of tumour diseases.

10. Use of a complex as claimed in one of claims 1 to 3, for sterilising and/or disinfecting surfaces or liquids.

## Revendications

1. Complexe d'hypéricine respectivement d'un sel d'hypéricine et de polyvinylpyrrolidone (PVP), **caractérisé en ce que** la part de masse moyenne d'hypéricine respectivement de sel d'hypéricine par rapport au complexe entier est supérieure à 6 % en masse.

2. Complexe selon la revendication 1, **caractérisé en ce que** le rapport moyen de quantité de matière d'hypéricine à la PVP dans le complexe est supérieur à 2,5.

3. Complexe selon la revendication 1 ou 2, **caractérisé en ce que** la PVP présente une masse molaire moyenne de 10 à 40 kD, de préférence de 12 à 25 kD.

4. Composition pharmaceutique contenant un complexe selon l'une des revendications 1 à 3.

5. Composition pharmaceutique selon la revendication 4, **caractérisée en ce que** la composition est prévue pour une administration par voie intraveineuse et **en ce que** la composition contient de l'hypéricine dans une concentration d'au moins 25 mg/L.

6. Procédé pour la préparation d'un complexe selon l'une des revendications 1 à 3, caractérisé en qu'un mélange d'hypéricine et PVP est chauffé à une température qui est supérieure à la température de transition vitreuse de la PVP utilisée.

7. Procédé selon la revendication 6, **caractérisé en ce que** le mélange d'hypéricine et PVP est additionné d'un solvant ou d'un mélange de solvants, de préférence eau, éthanol, méthanol, pyridine, acétone, éthylméthylcétone, et/ou acétate d'éthyle, encore mieux eau, éthanol, méthanol, et/ou pyridine, bien mieux encore eau et/ou éthanol.

8. Procédé selon la revendication 6 ou 7, **caractérisé en ce que** le mélange est maintenu pendant au moins 5 minutes à une température qui est supérieure à la température de transition vitreuse de la PVP utilisée.

9. Composition pharmaceutique selon la revendication 4 ou 5 pour l'utilisation dans un procédé thérapeutique, de préférence pour une application dans la thérapie photodynamique (PDT) destinée au traitement de maladies tumorales.

10. Utilisation d'un complexe selon l'une des revendications 1 à 3 pour la stérilisation et/ou désinfection de surfaces ou liquides.
